# FASCICULE DE BREVET EUROPEEN

(11) **EP 1 543 801 B1**
(45) Date de publication et mention de la délivrance du brevet: **08.08.2007**
(21) Numéro de dépôt: 04356199.2
(22) Date de dépôt: 17.12.2004
(51) Int. Cl.: A61F 2/32, A61F 2/40

(54) **Prothèse d'épaule ou de hanche et son procédé de montage**
Schulter- oder Hüftprothese und ihr Montageverfahren
Shoulder or hip prostheses and method of mounting it

(30) Priorité: 19.12.2003 FR 0315069
(43) Date de publication de la demande: 22.06.2005
(73) Titulaire: TORNIER, 38330 Saint-Ismier (FR)
(72) Inventeur: Tornier, Alain, 38330 Saint Ismier (FR)
(74) Mandataire: Myon, Gérard Jean-Pierre

(56) Documents cités:
- EP-A- 1 380 274
- CH-A- 426 096
- SU-A- 749 392
- US-A- 3 992 726
- US-A- 4 846 840

## Description

L'invention a trait à une prothèse totale ou partielle d'épaule ou de hanche permettant de reproduire, avec un degré de précision amélioré, les caractéristiques des articulations naturelles. L'invention a également trait à un procédé de montage d'une telle prothèse.

Dans le domaine des prothèses d'épaule, il est connu, par exemple de EP-A-0 299 889, de créer une surface articulaire convexe sur un composant glénoïdien, alors qu'une surface articulaire concave, de forme correspondante est formée sur un composant huméral. Le composant glénoïdien d'une telle surface est très invasif et il peut se produire un conflit sous-acrominal du composant huméral en fin de mouvement d'abduction.

Il est par ailleurs connu de US-A-4,846,840, qui est considéré comme représentatif de l'état de la technique le plus proche, de réaliser, sur un élément intermédiaire d'une prothèse, deux surfaces convexes globalement concentriques en vue de leur articulation sur des surfaces concaves de forme correspondante, prévues respectivement sur deux os à articuler l'un sur l'autre. Une telle prothèse est instable dans la mesure où aucun moyen n'est prévu pour éviter que les deux os coopérant avec l'élément intermédiaire ne s'écartent l'un de l'autre. En cas de défaillance des ligaments articulaires, une luxation ne peut être exclue.

C'est à ces inconvénients qu'entend plus particulièrement remédier l'invention en proposant une prothèse articulaire d'épaule ou de hanche qui reproduit l'articulation anatomique, tout en facilitant l'abduction du bras ou de la jambe, en l'absence de la coiffe des rotateurs pour l'épaule ou des structures stabilisatrices de la hanche.

Dans cet esprit, l'invention concerne une prothèse d'épaule ou de hanche comprenant un composant huméral ou fémoral présentant une surface d'articulation concave et un composant intermédiaire présentant une première et une seconde surface d'articulation convexes destinées à coopérer respectivement avec la surface d'articulation concave du composant huméral ou fémoral et avec une surface d'articulation glénoïdienne ou cotyloïdienne concave naturelle ou appartenant à un composant glénoïdien ou cotyloïdien. Cette prothèse est caractérisée en ce que la surface d'articulation concave du composant huméral ou fémoral est formée par un patin relié par un col à une partie de ce composant apte à être ancrée dans le canal médullaire huméral ou fémoral, en ce que le composant intermédiaire est pourvu d'un organe de retenue du composant huméral ou fémoral dans une position où le patin est en appui contre la première surface convexe du composant intermédiaire, en ce que cet organe de retenue définit un passage non circulaire dans lequel est apte à être déplacé le col précité, en fonction des mouvements du composant huméral ou fémoral par rapport aux autres composants de la prothèse, et en ce que cet organe de retenue définit avec la première surface d'articulation convexe du composant intermédiaire un volume de réception d'une partie du patin qui fait saillie radialement par rapport au col.

Grâce à l'invention, l'organe de retenue contribue à la stabilité de la prothèse, alors que le caractère non-circulaire du passage dans lequel est déplacé le col permet d'envisager des mouvements d'abduction de grande amplitude.

Selon un premier mode de réalisation de l'invention, l'organe de retenue est globalement en forme de U, de sorte que le passage qu'il définit débouche sur un côté de cet organe.

Selon d'autres modes de réalisation de l'invention, l'organe de retenue peut être en forme de bague rapportée sur le composant intermédiaire, avec une ouverture centrale qui constitué le passage non circulaire. Cette ouverture peut être de forme globalement elliptique ou de forme globalement rectangulaire. Le patin est alors avantageusement à section transversale non circulaire, notamment de forme globalement elliptique ou rectangulaire.

Le volume de réception défini entre l'organe de retenue et la première surface d'articulation convexe du composant intermédiaire peut avoir une épaisseur non constante autour d'un axe médian de la surface convexe du composant intermédiaire qui reçoit en appui le patin. De même, l'épaisseur de la partie du patin qui fait saillie radialement par rapport au col peut ne pas être constante autour d'un axe central de ce col.

L'organe de retenue peut être monté de façon amovible sur une partie principale du composant intermédiaire, notamment au moyen d'un organe élastique de blocage, tel qu'un circlip. En variante, l'organe de retenue peut être monobloc avec le composant intermédiaire. Il peut, selon une autre variante, être formé de plusieurs parties.

Le composant huméral ou fémoral peut être bipartite, la partie apte à être ancrée dans le canal médullaire huméral ou fémoral définissant un logement de réception d'un pion solidaire du col et du patin. Ce pion peut être centré sur un axe décalé par rapport à un axe longitudinal du col. Dans ce cas, on peut prévoir que le logement peut recevoir le pion dans au moins deux positions dans lesquelles l'axe central du pion est aligné avec un axe central du logement, la position angulaire de l'axe longitudinal du col par rapport à l'axe central du logement étant alors différente dans ces positions.

L'invention concerne également un procédé de montage d'une prothèse telle que précédemment décrite et, plus spécifiquement, un procédé qui comprend des étapes consistant à :
- introduire une tige appartenant au composant huméral ou fémoral dans un passage non circulaire défini par un organe de retenue appartenant au composant intermédiaire ;
- déplacer un ensemble, constitué de la tige, de l'organe précité et d'un patin qui forme la surface d'articulation concave du composant huméral ou fémoral, jusqu'à ce que ce patin soit en appui contre la première surface d'articulation convexe du composant intermédiaire et
- immobiliser l'organe précité par rapport à une partie principale du composant intermédiaire en retenant une partie du patin dans cette première surface convexe.

En particulier dans le cas où l'organe de retenue est monobloc avec le composant intermédiaire, le procédé de montage de la prothèse comprend une étape consistant à introduire en force le patin du composant huméral ou fémoral dans le passage non circulaire défini par l'organe de retenue, de telle sorte que son col est engagé dans ce passage et qu'une partie du patin est retenue dans un volume de réception défini entre l'organe de retenue et la première surface convexe du composant intermédiaire.

L'invention sera mieux comprise et d'autres avantages de celle-ci apparaîtront plus clairement à la lumière de la description qui va suivre de quatre modes de réalisation d'une prothèse conforme à son principe, donnée uniquement à titre d'exemple et faite en référence aux dessins annexés dans lesquels :
- la figure 1 est une coupe sagittale de principe d'une prothèse d'épaule conforme à l'invention en place sur un patient, alors que le bras du patient est en position intermédiaire ;
- la figure 2 est une coupe analogue à la figure 1 mais à plus petite échelle, alors que le bras du patient est en position relevée par rapport à celle de la figure 1 ;
- la figure 3 est une coupe analogue à la figure 2, alors que le bras du patient est en position basse ;
- la figure 4 est une coupe selon la ligne IV-IV à la figure 1, la rondelle de blocage, le circlip, le composant glénoïdien et la glène étant omis pour la clarté du dessin ;
- la figure 5 est une vue en perspective éclatée d'une partie du composant huméral de la prothèse des figures 1 à 4 ;
- la figure 6 est coupe sagittale partielle d'une prothèse de hanche conforme à un second mode de réalisation de l'invention, alors que la jambe du patient est en position intermédiaire ;
- la figure 7 est une coupe selon la ligne VII-VII à la figure 6, le composant fixe et l'os iliaque étant omis pour la clarté du dessin ;
- la figure 8 est une coupe selon la ligne VIII-VIII à la figure 7 ;
- la figure 9 est une coupe analogue à la figure 7 pour une prothèse d'épaule conforme à un troisième mode de réalisation de l'invention ;
- la figure 10 est une vue en perspective éclatée d'une partie du composant huméral de la prothèse de la figure 9 ;
- la figure 11 est une vue en perspective éclatée du composant huméral d'une prothèse d'épaule conforme à un quatrième mode de réalisation de l'invention et
- la figure 12 est une vue de détail dans le sens de la flèche XII à la figure 11.

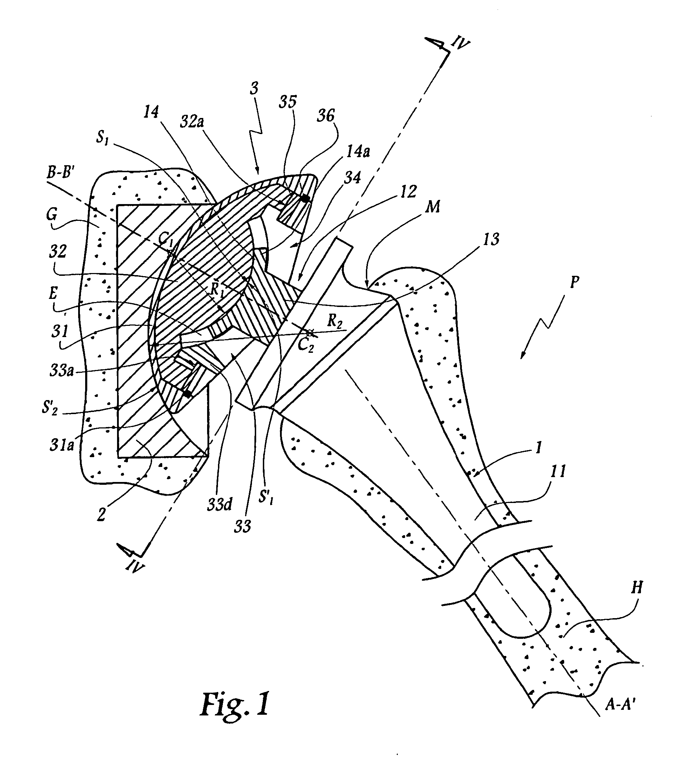

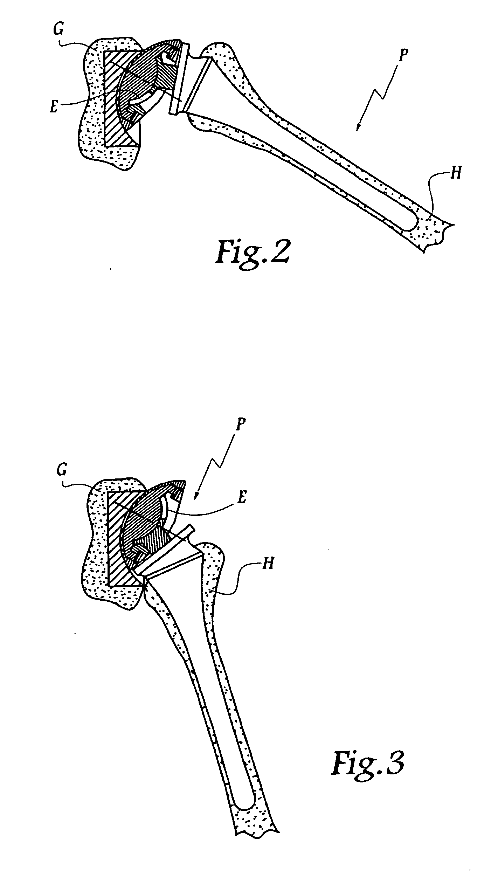

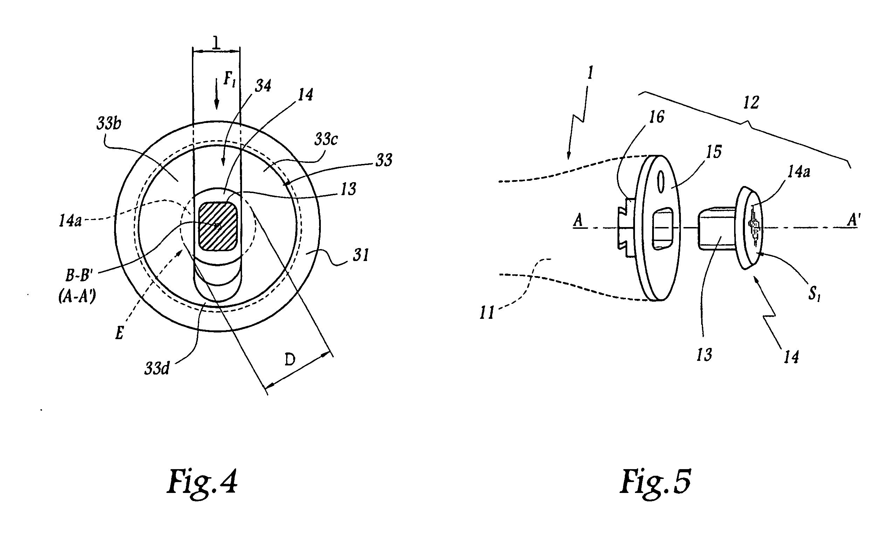

La prothèse P représentée sur la figure 1 à 5 comprend un composant huméral 1 qui inclut une partie 11 en forme de tige destinée à être ancrée dans le canal médullaire M de l'humérus H de l'articulation à équiper de la prothèse P. Un sous-ensemble 12 est immobilisé sur la partie 11 par coopération de formes. Ce sous-ensemble comprend un col 13 en forme de tige et un patin 14 qui fait saillie radialement par rapport au col autour d'un axe central A-A' au col. Le col est à section globalement rectangulaire, ainsi que cela ressort de la figure 4. Le sous-ensemble 12 comprend également une collerette 15 sur laquelle peut être immobilisé le col et qui est pourvue d'une queue d'aronde 16 permettant son blocage sur la partie 11. Tout autre moyen de blocage peut être utilisé ici.

Le col 13 et le patin 14 sont monobloc. Ils pourraient également être formés par deux pièces distinctes assemblées.

Le patin 14 définit une surface concave S₁ dont la concavité est tournée vers la glène G de l'épaule.

La prothèse comprend également un composant glénoïdien 2 ancré dans la glène G et définissant une surface concave S₂ dont la concavité est tournée vers l'extérieur de la glène.

Entre les composants 1 et 2 est intercalé un composant intermédiaire 3 comprenant une coupelle creuse 31 à l'intérieur de laquelle sont immobilisés un bouton ou insert 32 et un organe 33 globalement en forme de U ainsi que cela ressort de la figure 4. On note 34 l'ouverture prévue entre les deux branches de l'organe 33.

Les éléments 32 et 33 sont maintenus en position dans la coupelle 31 au moyen d'une rondelle 35 qui vient en appui contre deux surfaces annulaires 32a et 33a prévues respectivement sur le bouton 32 et sur l'organe 33. Cette rondelle 35 est maintenue en position par rapport à la coupelle 31 au moyen d'un circlip 36 engagé dans une rainure interne 31a de la coupelle 31.

On note S'₁ la surface convexe du bouton 32 accessible depuis l'extérieur de la coupelle 31. Les surfaces S₁ et S'₁ sont toutes deux des tronçons de sphère et ont sensiblement le même rayon R₁, de telle sorte que le patin 14 peut glisser sur la surface S'₁ du bouton 32.

La surface extérieure convexe S'₂ de la coupelle 31 est également en forme de tronçon de sphère, avec un rayon R₂ analogue au rayon de la surface S₂, ce qui permet un mouvement de glissement relatif des surfaces S₂ et S'₂.

Ainsi, l'articulation de l'humérus H par rapport à la glène G a lieu par glissement des surfaces S₁ et S'₁ l'une sur l'autre et des surfaces S₂ et S'₂ l'une sur l'autre.

Le centre de rotation C₁ des surfaces S₁ et S'₁ est situé dans le composant 2, alors que le centre de rotation C₂ des surfaces S₂ et S'₂ est situé à l'extérieur de la prothèse à l'opposé de la glène.

Pour éviter un déboîtement du patin 14, il est prévu que l'organe 33 forme un moyen de retenue du patin 14 au contact de la surface S'₁.

Pour ce faire, les branches 33b et 33c et le fond 33d de l'organe 33 définissent avec la surface S'₁ un espace E dans lequel peut être engagée la partie 14a du patin 14 qui fait saillie, par rapport au col 13, radialement autour de l'axe A-A'. La largeur l de l'ouverture 34 est inférieure au diamètre D du patin 14 qui est circulaire. Ainsi, dès que le col 13 est en place dans l'ouverture 34, la partie 14a est engagée dans l'espace E et les composants 1 et 2 ne peuvent plus être écartés l'un de l'autre tant que la l'organe 33 est immobilisé sur la coupelle 31.

Sur la figure 4, la rondelle 35 et le circlip 36 ne sont pas représentés, ce qui correspond à une étape intermédiaire d'assemblage de la prothèse P selon un premier procédé.

A la figure 4, l'ouverture 34 débouche vers le haut. Cependant, l'orientation de l'organe 33 peut être choisie pour que l'ouverture 34 débouche dans n'importe quelle direction sur cette figure, en fonction de l'orientation du mouvement dont on souhaite favoriser l'amplitude.

Pour le montage de la prothèse, l'organe 33 est engagé sur le col 13 avant que le patin 14 ne soit plaqué sur la surface S'₁. Cette opération est aisée compte tenu de la forme en U de l'organe 33, ses branches 33b et 33c étant alors disposées de part et d'autre de la tige 13. On peut alors déplacer l'ensemble formé des éléments 13, 14 et 33 vers le bouton 32 sans être limité par la taille de l'ouverture 34 puisque l'organe 33 est alors en arrière de la partie 14a par rapport à son sens de déplacement. Ensuite, lorsque le patin 14 est au contact du bouton 32, il suffit d'immobiliser l'organe 33 sur la coupelle 31 au moyen de la rondelle 35 et du circlip 36.

On note B-B' l'axe médian de la surface S'₁ qui est confondu avec l'axe A-A' dans la représentation de la figure 4. Le fait que l'ouverture 34 n'est pas symétrique autour de l'axe B-B' permet une mise en place aisée et une retenue efficace du patin 14, et par là même de l'ensemble du composant 1, par rapport au composant 3.

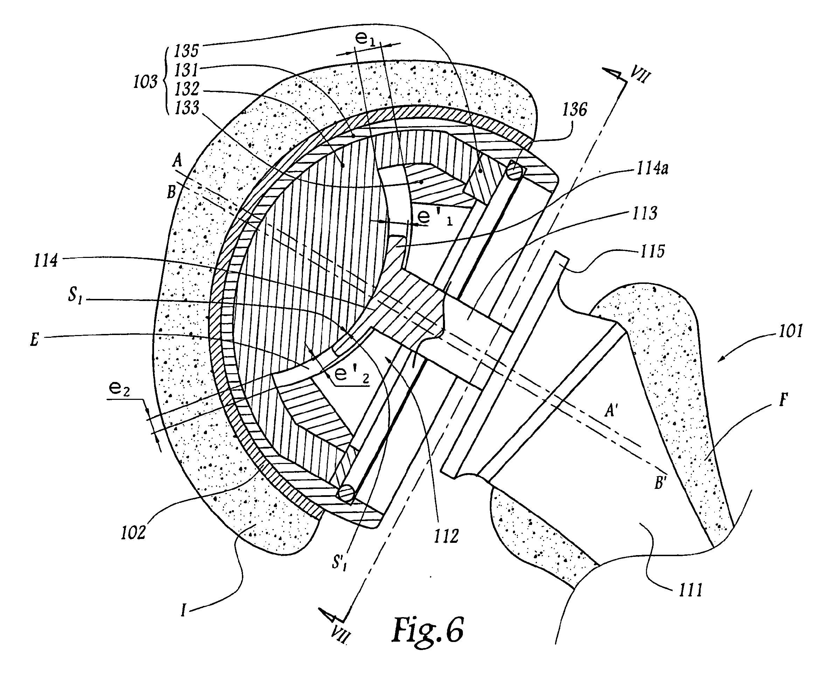

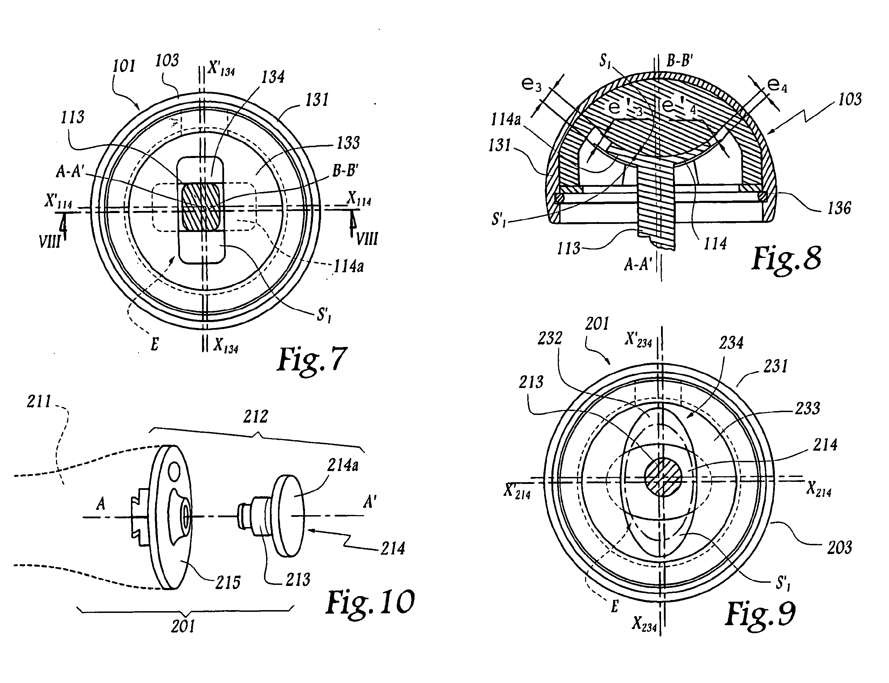

Dans le second mode de réalisation représenté aux figure 6 et 7, les éléments analogues à ceux du premier mode de réalisation portent des références identiques augmentées de 100. Le composant intermédiaire 103 de ce mode de réalisation comprend une coupelle 131 et un bouton 132 qui définit une surface S'₁ d'articulation d'un patin 114 appartenant à un composant fémoral 101. Le patin 114 est monobloc avec un col 113 avec lequel il constitue un sous-ensemble 112 destiné à être monté sur une partie 111 ancrée dans le canal médullaire du fémur F. Le composant fixe 102 est ancré dans l'os iliaque I.

Une bague annulaire 133 est montée dans la coupelle 131 en tant qu'organe de retenue du patin 114. L'ouverture centrale 134 de la bague 133 est globalement rectangulaire, avec son plus grand axe X₁₃₄-X'₁₃₄ globalement parallèle au plan sagittal.

Le patin 114 est également globalement rectangulaire, avec son plus grand axe X₁₁₄-X'₁₁₄ orienté perpendiculairement au plan sagittal dans la configuration de la figure 7.

On note S₁ la surface d'articulation formée par le patin 114.

Comme précédemment, un espace E est défini entre la bague 133 et le bouton 132 pour la réception d'une partie 114a du bouton 114 qui fait saillie radialement par rapport à la tige 113.

Comme il ressort plus particulièrement des figures 6 et 8, l'épaisseur e₁ de l'espace E dans sa partie supérieure à la figure 6 est plus importante que l'épaisseur correspondante e₂ dans la partie inférieure. De la même façon, l'épaisseur e₃ de l'espace E sur la gauche de la figure 8 est supérieure à l'épaisseur correspondante e₄ sur la droite de cette figure. En d'autres termes, l'espace E est d'épaisseur non constante autour de l'axe central médian B-B' de la surface S'₁.

En outre, la partie 114a du patin 114 qui fait saillie radialement par rapport au col 113 est d'épaisseur également non constante autour de l'axe A-A' du col 113, l'épaisseur e'₁ de la portion de cette partie 114a destinée à être introduite dans la partie de l'espace E d'épaisseur e₁ étant plus importante que celle e'₂ de la portion de la partie 114a destinée à être introduite dans la partie de l'espace E d'épaisseur e₂. De la même façon, la portion de la partie 114a destinée à être introduite dans la partie de l'espace E d'épaisseur e₃ a une épaisseur e'₃ plus importante que l'épaisseur e₄' de la portion de la partie 114a destinée à être introduite dans la partie de l'espace E d'épaisseur e₄.

Avec cette répartition des épaisseurs respectives de l'espace E et de la partie 114a du patin 114, on obtient un contrôle de la position angulaire du col 113, et par là même, de l'ensemble du composant 1, autour de l'axe central A-A' du col 113.

Ce contrôle évite que le patin 114 n'ait tendance à revenir dans une configuration où il pourrait être arraché à travers l'ouverture 134 de la bague 133.

Le montage de la prothèse P de ce mode de réalisation a lieu en désolidarisant le col 113 de la collerette 115 du sous-ensemble 112. Ceci permet d'introduire le col 113 dans l'ouverture 134 puis de solidariser à nouveau le col 113 et la collerette 115 par tout moyen approprié. Il est alors possible de plaquer le patin 114 contre le bouton 132 en amenant la bague 133 au contact du bouton 132 et la coupelle 131. Cette bague peut alors être immobilisée par rapport aux éléments 131 et 132 par mise en place d'une rondelle d'appui 135 et d'un circlip 136.

Lorsqu'on amène la bague 133 au contact du bouton 132, on oriente le patin 114 de telle façon que sa plus grande dimension soit globalement perpendiculaire à la plus grande dimensions de l'ouverture 134 en veillant à faire respectivement coïncider les zones d'épaisseur e' ₁, e'₂, e' ₃ et e'₄ avec les parties de l'organe 133 destinées à définir les zones de l'espace E d'épaisseurs e₁, e₂, e₃ et e₄. Ainsi, une fois la bague 133 plaquée sur le composant 103, le patin 114 est nécessairement orienté dans la configuration des figures 6 à 8 et ne risque pas de revenir accidentellement vers une configuration où il pourrait être extrait à travers l'ouverture 134, notamment dans le cas où ses dimensions sont inférieures à celles de l'ouverture.

Comme dans le premier mode de réalisation, le patin 114 est retenu en appui sur la surface S'₁ tout au long du mouvement d'abduction, y compris en position centrale correspondant à celle de la figure 1.

Dans le troisième mode de réalisation de l'invention représenté aux figures 9 et 10, les éléments analogues à ceux du premier mode de réalisation portent des références identiques augmentées de 200. Ce mode de réalisation concerne une prothèse d'épaule dont le composant intermédiaire 203 comprend une coupelle 231 et une bague 233 dont l'ouverture centrale 234 est de forme globalement elliptique, avec son axe longitudinal X₂₃₄-X'₂₃₄ globalement parallèle au plan sagittal.

Le sous-ensemble 212 du composant huméral 201, qui forme une surface d'articulation S₁ apte à coopérer avec la surface d'articulation S'₁ définie par le bouton 232 du composant 203, est plus particulièrement visible à la figure 9. Son patin 214 est de forme globalement elliptique, son col 213 étant circulaire. Le col 213 est apte à être immobilisé sur une collerette 215 dont il peut être séparé, ainsi que cela ressort des explications qui suivent.

On note 214a la partie du patin 214 qui fait saillie radialement par rapport au col 213. Comme précédemment la partie 214a du patin 214 est d'épaisseur non constante autour de l'axe A-A' du col 213, alors que l'espace E défini entre les éléments 233 et 232 pour recevoir cette partie est également d'épaisseur non constante. Par exemple, la partie de l'espace E représentée sur la gauche de la figure 10 peut avoir une épaisseur relativement faible, de même que la section correspondante de la partie 214a, alors que l'espace E à une épaisseur plus importante sur la droite de la figure 10, de même que la section correspondante de la partie 214a. Cette différence d'épaisseur contribue au maintien en position du patin 214 par rapport au composant 103 en rotation autour de l'axe B-B' .

L'assemblage de la prothèse conforme à ce mode de réalisation a lieu d'une façon analogue à celle du second mode de réalisation. On désolidarise le col 213 de la collerette 215 et on introduit ce col dans l'ouverture 234 de la rondelle 233. On applique alors le patin 214 contre la surface S'₁ du composant intermédiaire en respectant une orientation compatible avec les différentes épaisseurs de la partie 214a et de l'espace E, ce qui permet d'amener la rondelle 233 au contact de la coupelle 231 et de l'immobiliser par tout moyen approprié, notamment une rondelle du type de la rondelle 135 du second mode de réalisation.

Le col 213 et la collerette 215 peuvent être solidarisés avant ou après que la patin 214 soit plaqué contre la surface S'₁. De même en est-il en ce qui concerne le second mode de réalisation.

Selon une variante non représentée de l'invention, la collerette 215 et la partie 211 destinée à être ancrée dans le canal médullaire sont monoblocs. Selon une autre variante, le col 213 est introduit directement dans un logement formé par la partie 211.

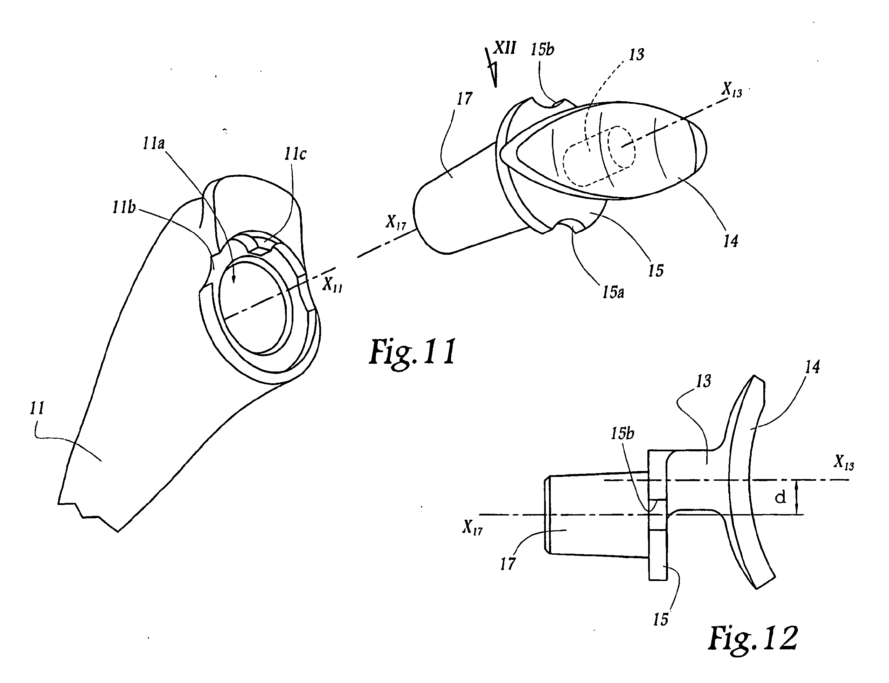

Dans le quatrième mode de réalisation de l'invention représenté à la figure 11, les éléments analogues à ceux du premier mode de réalisation portent des références identiques. La tige médullaire 11 définit un logement tronconique 11a centré sur un axe X₁₁ et bordé par une surface de portée 11b globalement annulaire. Le patin 14 et le col 13 sont mono-bloc avec la collerette 15 et avec un pion 17 destiné à être introduit dans le logement 11a et immobilisé dans celui-ci par coopération de formes. L'ensemble formé des éléments 13, 14, 15 et 17 est visible en vue de dessus à la figure 12. La surface externe du pion 17 est tronconique avec le même angle de conicité que la surface définissant le logement 11a.

On note X₁₇ l'axe central du pion 17. Les axes X₁₁ et X₁₇ sont confondus lorsque le pion 17 est reçu dans le logement 11a. On note X₁₃ l'axe central du col 13. L'axe X₁₃ est décalé radialement par rapport à l'axe X₁₇ d'une distance d non nulle, ce qui permet d'ajuster la position du patin 14 par rapport au composant intermédiaire non représenté de ce mode de réalisation.

La collerette 15 est destinée à venir en appui sur la surface 11b et pourvue de deux encoches 15a et 15b destinées à être disposées au niveau d'une saillie 11c formée au-dessus de la surface annulaire 11b, de telle sorte que l'ensemble formé des éléments 13, 14, 15 et 17 peut être monté sur la partie 11 dans deux positions, selon que la saillie 11c est reçue dans l'encoche 15a ou dans l'encoche 15b. On obtient ainsi deux positions possibles pour le patin 14 par rapport à la partie 11, du fait des deux positions angulaires obtenues pour l'axe X₁₃ par rapport aux axes X₁₁ et X₁₇.

Selon une variante non représentée de l'invention, les axes X₁₇ et X₁₃ peuvent être alignés.

Dans la description ci-dessus on a mentionné le positionnement du patin par rapport au composant intermédiaire, ceci est un positionnement relatif et l'assemblage peut avoir lieu en maintenant immobile le patin huméral ou fémoral et en déplaçant le composant intermédiaire.

L'invention a été représentée avec des organes de retenue 33, 133 ou 233 rapportés sur le composant intermédiaire 3, 103 ou 203. Elle est cependant applicable avec un organe de retenue monobloc avec le composant intermédiaire. Dans ce cas, le patin est introduit en force à travers le passage de l'organe de retenue lors du montage de la prothèse, ce qui permet d'engager le col correspondant dans ce passage. Un tel montage en force est également envisageable avec les prothèses des modes de réalisations représentés.

Selon une autre variante non représentée de l'invention, l'organe de retenue peut être bi-partite, voire multi-partite.

Indépendamment du mode de réalisation considéré, le composant intermédiaire peut être entièrement en matériau synthétique, notamment en polyéthylène, ou entièrement en céramique. Ceci n'est cependant pas obligatoire, ainsi qu'il ressort des figures.

On comprend que les procédés de montage mentionnés ci-dessus peuvent faire partie d'une méthode de pose d'une prothèse conforme à l'invention, la tige 11, 111 ou équivalente du composant huméral ou fémoral étant tout d'abord ancrée dans le canal médullaire correspondant, l'assemblage avec le composant intermédiaire ayant lieu ensuite.

L'invention a été représentée lors de sa mise en oeuvre avec des prothèses totales d'épaule et de hanche. Elle est cependant applicable avec une prothèse d'épaule dépourvue de composant glénoïdien, la surface concave de la glène étant utilisée au lieu de la surface S₂ représentée sur les figures. De même en est-il dans le cas d'une prothèse de hanche où la cavité cotyloïde naturelle peut être utilisée.

Les caractéristiques des différents modes de réalisation représentées peuvent être combinées entre elles dans le cadre de la présente invention. En particulier, la prothèse du second mode de réalisation peut être adaptée à l'épaule, alors que les prothèses des premier et troisième mode de réalisation peuvent être adaptées à la hanche.

## Revendications

1. Prothèse (P) d'épaule ou de hanche comprenant un composant huméral ou fémoral (1, 101, 201) présentant une surface d'articulation concave (S₁) et un composant intermédiaire (3; 103; 203) présentant une première et une seconde surfaces d'articulation convexes (S'₂, S'₂ ) destinées à coopérer respectivement avec ladite surface d'articulation concave (S₁) dudit composant huméral ou fémoral (1; 101; 201) et avec une surface d'articulation glénoïdienne (S₂) ou cotyloïdienne concave naturelle ou appartenant à un composant glénoïdien ou cotyloïdien, **caractérisée en ce que** la surface d'articulation concave (S₁) dudit composant huméral ou fémoral (1 ; 101 ; 201) est formée par un patin (14 ; 114 ; 214) relié par un col (13 ; 113 ; 213) à une partie (11 ; 111) dudit composant apte à être ancrée dans le canal médullaire (M) huméral ou fémoral, **en ce que** ledit composant intermédiaire (3 ; 103 ; 203) est pourvu d'un organe (33 ; 133 ; 233) de retenue dudit composant huméral ou fémoral en appui contre la première surface convexe dudit composant intermédiaire, **en ce que** ledit organe de retenue (33 ; 133 ; 233) définit un passage non circulaire (34 ; 134 ; 234) dans lequel est apte à être déplacé ledit col (13 ; 113 ; 213) en fonction des mouvements dudit composant huméral ou fémoral par rapport aux autres composants (2, 3 ; 103 ; 203) de la prothèse, et **en ce que** ledit organe de retenue (33 ; 133 ; 233) définit avec ladite première surface d'articulation convexe (S'₁) dudit composant intermédiaire (3 ; 103 ; 203) un volume (E) de réception d'une partie (14a ; 114a ; 214a) dudit patin faisant saillie radialement par rapport audit col.

2. Prothèse selon la revendication 1, **caractérisée en ce que** ledit organe de retenue (33) est globalement en forme de U.

3. Prothèse selon la revendication 1, **caractérisée en ce que** ledit organe de retenue est en forme de bague (133 ; 233) rapportée sur ledit composant intermédiaire (103 ; 203) et dont l'ouverture centrale (134 ; 234) constitue ledit passage.

4. Prothèse selon la revendication 3, **caractérisée en ce que** ladite ouverture (134) est de forme globalement rectangulaire.

5. Prothèse selon la revendication 3, **caractérisée en ce que** ladite ouverture (234) est de forme globalement elliptique.

6. Prothèse selon l'une des revendications 3 à 5, **caractérisée en ce que** ledit patin (114 ; 214) est à section transversale non circulaire, notamment de forme globalement elliptique ou rectangulaire.

7. Prothèse selon l'une des revendications précédentes, **caractérisée en ce que** ledit volume de réception (E) est d'épaisseur (e₁, e₂, e₃, e₄) non constante autour d'un axe central (B-B') médian de ladite première surface d'articulation convexe (S'₁).

8. Prothèse selon l'une des revendications précédentes, **caractérisée en ce que** l'épaisseur (e'₁, e'₂, e'₃, e'₄) de la partie (114a ; 214a) dudit patin (114 ; 214) qui fait saillie radialement par rapport à audit col (113 ; 213) n'est pas constante autour d'un axe central (A-A') de ladite tige.

9. Prothèse selon l'une des revendications précédentes, **caractérisée en ce que** ledit organe de retenue (33 ; 133 ; 233) est monté de façon amovible sur une partie principale (31 ; 131 ; 231) dudit composant intermédiaire (3 ; 103 ; 203), notamment au moyen d'un organe élastique de blocage (36 ; 136).

10. Prothèse selon l'une des revendicaitons 1 à 8, **caractérisée en ce que** ledit organe de retenue est monobloc avec ledit composant intermédiaire.

11. Prothèse selon l'une des revendications précédentes, **caractérisée en ce que** ledit organe de retenue est formé d'au moins deux parties.

12. Prothèse selon l'une des revendications précédentes, **caractérisée en ce que** ledit composant huméral ou fémoral est bipartite, ladite partie (11 ; 211) apte à être ancrée dans le canal médullaire huméral ou fémoral définissant un logement (11a) de réception d'un pion (17) solidaire dudit col (13 ; 213) et dudit patin (14 ; 214).

13. Prothèse selon la revendication 12, **caractérisée en ce que** ledit pion (17) est centré sur un axe (X₁₇) décalé (d) par rapport à un axe longitudinal (X₁₃) dudit col (13).

14. Prothèse selon la revendication 13, **caractérisée en ce que** ledit logement (11a) est apte à recevoir ledit pion (17) dans au moins deux positions dans lesquelles l'axe central (X₁₇) dudit pion est aligné avec un axe central (X₁₁) dudit logement (11a), la position angulaire de l'axe longitudinal (X₁₃) dudit col (13) par rapport à l'axe central (X₁₁) dudit logement étant différente dans lesdites deux positions.

15. Procédé de montage d'une prothèse d'épaule ou de hanche selon la revendication 1 comprenant un composant huméral ou fémoral présentant une surface d'articulation concave et un composant intermédiaire présentant une première et une seconde surfaces d'articulation convexes destinées à coopérer respectivement avec ladite surface d'articulation concave dudit composant huméral ou fémoral et avec une surface d'articulation glénoïdienne ou cotyloïdienne concave naturelle ou appartenant à un composant glénoïdien ou cotyloïdien, **caractérisé en ce qu'**il comprend des étapes consistant à :
- introduire un col (13 ; 113 ; 213) appartenant audit composant huméral (1 ; 201) ou fémoral (101) dans un passage (34 ; 134 ; 234) non circulaire défini par un organe (33 ; 133 ; 233) destiné à faire partie dudit composant intermédiaire (3 ; 103 ; 203) ;
- déplacer un ensemble, formé dudit col, dudit organe et d'un patin (14 ; 114 ; 214) formant ladite surface d'articulation concave dudit composant huméral ou fémoral, jusqu'à ce que ledit patin soit en appui contre ladite première surface d'articulation convexe (S'₁) dudit composant intermédiaire et
- immobiliser ledit organe par rapport à une partie principale (31 ; 131 ; 231) dudit composant intermédiaire en retenant une partie (14a ; 114a ; 214a) dudit patin dans un volume de réception (E) défini entre ledit organe et ladite première surface convexe.

16. Procédé selon la revendication 15 pour le montage d'une prothèse selon la revendication 6, **caractérisée en ce que** lors du déplacement dudit ensemble (113, 114, 133, 213, 214, 233), on oriente ledit patin (114 ; 214) par rapport audit organe de retenue (133 ; 233) de telle sorte que sa plus grande dimension (X₁₁₄-X'₁₁₄ ; X₂₁₄-X'₂₁₄) est globalement perpendiculaire à la plus grande dimension (X₁₃₄-X'₁₃₄ ; X₂₃₄-X' ₂₃₄) dudit passage (134 ; 234).

17. Procédé selon la revendication 15 pour le montage d'une prothèse selon les revendications 7 et 8, **caractérisée en ce que** lors du déplacement dudit ensemble (113, 114, 133 ; 213, 214, 233), on oriente ledit patin (114 ; 214) par rapport audit organe de retenue (133 ; 233) de telle sorte que les zones de plus faibles épaisseur (e'₂, e'₄) de la partie (114a ; 214a) dudit patin qui fait saillie par rapport audit col (113 ; 213) coïncident respectivement avec les parties dudit organe destinées à définir les zones dudit volume de réception (E) de plus faibles épaisseur (e₁, e₂), alors que les zones de plus fortes épaisseurs (e'₃, e'₄) de ladite partie coïcident respectivement avec les parties dudit organe destinées à définir les zones dudit volume de plus fortes épaisseur (e₃, e₄).

18. Procédé de montage d'une prothèse d'épaule ou de hanche selon la revendication 1, ladite prothèse comprenant un composant huméral ou fémoral présentant une surface d'articulation concave et un composant intermédiaire présentant, une première et une seconde surfaces d'articulation convexes destinées à coopérer respectivement avec ladite surface d'articulation concave dudit composant huméral ou fémoral et avec une surface d'articulation glénoidienne ou cotyloïdienne concave naturelle ou appartenant à un composant glénoïdien ou cotyloïdien, **caractérisé en ce qu'**il comprend une étape consistant à introduire en force le patin ((14 ; 114 ; 214) dudit composant huméral (1; 201) ou fémoral (101) dans le passage (34 ; 134 ; 234) défini par ledit organe de retenue (33 ; 133 ; 233) de telle sorte que ledit col (13 ; 113 ; 213) est engagé dans ledit passage et qu'une partie (14a ; 114a ; 214a) dudit patin est retenue dans un volume (E) de réception défini entre ledit organe et la première surface convexe (S'₁) dudit composant intermédiaire (3 ; 103 ; 203) .

## Claims

1. Shoulder or hip prosthesis (P) comprising a humeral or femoral component (1; 101; 201) having a concave articulating surface (S₁) and an intermediate component (3; 103; 203) having a first and second convex articulating surface (S₆', S₂') designed to cooperate respectively with said concave articulating surface (S₁) of said humeral or femoral component (1; 101; 201) and with a natural concave glenoidal (S₂) or cotyloidal articulating surface or one belonging to a glenoidal or cotyloidal component, **characterised in that** the concave articulating surface (S₁) of said humeral or femoral component (1; 101; 201)is formed of a shoe (14; 114; 214) connected by a neck (13; 113; 213) to a part (11; 111) of said component capable of being anchored in the humeral or femoral medullary canal (M), **in that** said intermediate component (3; 103; 203) is provided with a device (33; 133; 233) for retaining said humeral or femoral component applied against the first convex surface of said intermediate component, **in that** said retaining device (33; 133; 233) defines a non-circular passage (34; 134; 234) in which said neck (13, 113; 213) can be moved according to the movements of said humeral or femoral component relative to the other components (2; 3; 103; 203) of the prosthesis, and **in that** said retaining device (3; 133; 233) defines with said first convex articulating surface (S'₁) of said intermediate component (3; 103; 203) a volume (E) for receiving a part (14a; 114a; 214a) of said shoe projecting radially relative to said neck.

2. Prosthesis according to claim 1, **characterised in that** said retaining device (33) is overall in the shape of a U.

3. Prosthesis according to claim 1, **characterised in that** said retaining device is in the shape of a ring (133; 233) added onto said intermediate component (103; 203) and of which the central opening (134; 234) constitutes said passage.

4. Prosthesis according to claim 3, **characterised in that** said opening (134) is overall of a rectangular shape.

5. Prosthesis according to claim 3, **characterised in that** said opening (234) is overall of an elliptical shape.

6. Prosthesis according to one of claims 3 to 5, **characterised in that** said shoe (114; 214) has a non-circular cross section, in particular with an overall elliptical or rectangular shape.

7. Prosthesis according to one of the preceding claims, **characterised in that** said receiving volume (E) has a non-constant thickness (e₁, e₂, e₃, e₄) about a middle central axis (B-B') of said first convex articulating surface (S'₁).

8. Prosthesis according to one of the preceding claims, **characterised in that** the thickness (e'₁, e'₂, e'₃, e'₄) of the part (114a; 214a) of said shoe (114;214) which projects radially relative to said neck (113;213) is not constant about a central axis (A-A') of said stem.

9. Prosthesis according to one of the preceding claims **characterised in that** said retaining device (33; 133; 233) is mounted in a detachable manner on a main part (31; 131; 231) of said intermediate component (3; 103; 203) in particular by means of an elastic locking component (36; 136).

10. Prosthesis according to one of claims 1 to 8 **characterised in that** said retaining device is made in one piece with said intermediate component.

11. Prosthesis according to one of the preceding claims, **characterised in that** said retaining device is formed of at least two parts.

12. Prosthesis according to one of the preceding claims, **characterised in that** said humeral or femoral component is bipartite, said part (11; 211) capable of being anchored in the humeral or femoral medullary canal defining a housing (11a) for receiving a pin (17) secured to said neck (13; 213) and said shoe (14; 214).

13. Prosthesis according to claim 12, **characterised in that** said pin (17) is centred on an axis (X₁₇) offset (d) relative to a longitudinal axis (X₁₃) of said neck (13).

14. Prosthesis according to claim 13, **characterised in that** said housing (11a) is capable of receiving said pin (17) in at least two positions in which the central axis (X₁₇) of said pin is aligned with a central axis (X₁₁) of said housing (11a), the angular position of the longitudinal axis (X₁₃) of said neck (13) relative to the central axis (X₁₁) of said housing being different in said two positions.

15. Method for mounting a shoulder or hip prosthesis according to claim 1, comprising a humeral or femoral component having a concave articulating surface and an intermediate component having a first and second convex articulating surface designed to cooperate respectively with said concave articulating surface of said humeral or femoral component and with a natural concave glenoidal or cotyloidal articulating surface or one belonging to a glenoidal or cotyloidal component, **characterised in that** it comprises steps consisting of:
- introducing a neck (13; 113; 213) belonging to said humeral component (1; 201) or femoral component (101) into a non-circular passage (34; 134; 234) defined by a device (33; 113; 233) designed to form part of said intermediate component (3; 103; 203);
- moving an assembly, formed of said neck, said device and a shoe (14; 114; 214) forming said concave articulating surface of said humeral or femoral component, until said shoe rests against said first convex articulating surface (S'₂) of said intermediate component; and
- immobilising said device relative to a main part (31; 131; 231) of said intermediate component by retaining a part (14a; 114a; 214a) of said shoe in a receiving volume (E) defined between said device and said first convex surface.

16. Method according to claim 15 for mounting a prosthesis according to claim 6, **characterised in that** when said assembly (113, 114, 133, 213, 214, 233) is moved, said shoe (114; 214) is oriented relative to said retaining device (133; 213) so that the largest dimension (X₁₁₄-X'_{114;} X₂₁₄-X'₂₁₄) is perpendicular overall to the largest dimension (X₁₃₄-X'_{134;} X₂₃₄-X'₂₃₄) of said passage (134; 234).

17. Method according to claim 15 for mounting a prosthesis according to claims 7 and 8, **characterised in that** when said assembly (113, 114, 133; 213, 214, 233) is moved, said shoe (114; 214) is oriented relative to said retaining device (133; 233) so that the zones with the smallest thickness (e'₂, e'₄) of the part (114a ; 214a) of said shoe which projects relative to said neck (113; 213) coincide respectively with the parts of said component designed to define the zones of said receiving volume (E) with the smallest thickness (e₁, e₂), while the zones with the largest thickness (e'₃, e'₄) of said part coincide respectively with the parts of said device designed to define the zones of said volume with the largest thickness (e₃, e₄).

18. Method for mounting a shoulder or hip prosthesis according to claim 1, said prosthesis comprising a humeral or femoral component having a concave articulating surface and an intermediate component having a first and second convex articulating surface designed to cooperate respectively with said concave articulating surface of said humeral or femoral component and with a natural concave glenoidal or cotyloidal articulating surface or one belonging to a glenoidal or cotyloidal component, **characterised in that** it comprises a step consisting of introducing by force the shoe (14; 114; 214) of said humeral component (1; 201) or femoral component (101) into the passage (34; 134; 234) defined by said retaining device (33; 133; 233) so that said neck (13; 113; 213) is engaged in said passage and that a part (14a ; 114a ; 214a) of said shoe is retained in a receiving volume (E) defined between said device and the first convex surface (S'₁) of said intermediate component (3; 103; 203).

## Patentansprüche

1. Schulter- oder Hüftprothese (P), umfassend eine Humerus- oder Femurkomponente (1; 101; 201), die eine konkave Artikulationsoberfläche (S₁) aufweist, und eine Zwischenkomponente (3; 103; 203) mit einer ersten und einer zweiten konvexen Artikulationsoberfläche (S'₁, S'₂), die dazu bestimmt sind, jeweils mit der konkaven Artikulationsoberfläche (S₁) der Humerus- oder Femurkomponente (1; 101; 201) und mit einer konkaven Glenoid- (S₂) oder Acetabulum-Artikulationsoberfläche zusammenzuwirken, die natürlich ist oder sich an einer Glenoid- oder Acetabulumkomponente befindet, **dadurch gekennzeichnet, dass** die konkave Artikulationsoberfläche (S₁) der Humerus- oder Femurkomponente (1; 101; 201) von einer Gleitkufe (14; 114; 214) gebildet wird, die durch einen Hals (13; 113; 213) mit einem Teil (11; 111) der Komponente verbunden ist, der derart ausgelegt ist, dass er im Markkanal (M) des Humerus oder Femurs verankert werden kann, **dadurch**, dass die Zwischenkomponente (3; 103; 203) mit einem Organ (33; 133; 233) zum Zurückhalten der Humerus- oder Femurkomponente in der Auflage gegen die erste konvexe Oberfläche der Zwischenverbindung ausgestattet ist, **dadurch**, dass das Zurückhalteorgan (33; 133; 233) einen nicht-runden Durchgang (34; 134; 234) definiert, in den der Hals (13; 113; 213) in Abhängigkeit von Bewegungen der Humerus- oder Femurkomponente in Bezug auf die anderen Komponenten (2, 3; 103; 203) der Prothese verlagert werden kann, und **dadurch**, dass das Zurückhalteorgan (33; 133; 233) mit der ersten konvexen Artikulationsoberfläche (S'₁) der Zwischenkomponente (3; 103; 203) ein Volumen (E) zum Aufnehmen eines Teils (14a; 114a; 214a) der Gleitkufe definiert, das in Bezug auf den Hals radial hervorspringt.

2. Prothese nach Anspruch 1, **dadurch gekennzeichnet, dass** das Zurückhalteorgan (33) im Wesentlichen U-förmig ist.

3. Prothese nach Anspruch 1, **dadurch gekennzeichnet, dass** das Zurückhalteorgan die Form eines in die Zwischenkomponente (103; 203) eingesetzten Rings (133; 233) hat, dessen zentrale Öffnung (134; 234) den Durchgang bildet.

4. Prothese nach Anspruch 3, **dadurch gekennzeichnet, dass** die Öffnung (134) eine im Wesentlichen rechteckige Form hat.

5. Prothese nach Anspruch 3, **dadurch gekennzeichnet, dass** die Öffnung (234) eine im Wesentlichen elliptische Form hat.

6. Prothese nach einem der Ansprüche 3 bis 5, **dadurch gekennzeichnet, dass** die Gleitkufe (114; 214) einen nicht-runden, insbesondere einen im Wesentlichen elliptisch oder rechteckig geformten, Querschnitt besitzt.

7. Prothese nach einem der vorhergehenden Ansprüche, **dadurch gekennzeichnet, dass** das Aufnahmevolumen (E) eine nicht konstante Dicke (e₁, e₂, e₃, e₄) um eine zentrale Mittelachse (B-B') der ersten konvexen Artikulationsoberfläche (S'₁) herum hat.

8. Prothese nach einem der vorhergehenden Ansprüche, **dadurch gekennzeichnet, dass** die Dicke (e'₁, e'₂, e'₃, e'₄) des Teils (114a, 214a) der Gleitkufe (114; 214), das in Bezug auf den Hals (113; 213) hervorragt, um eine zentrale Achse (A-A') des Schafts herum nicht konstant ist.

9. Prothese nach einem der vorhergehenden Ansprüche, **dadurch gekennzeichnet, dass** das Zurückhalteorgan (33; 133; 233), insbesondere mithilfe eines elastischen Blockierungsorgans (36; 136), unbeweglich an einem Hauptteil (31; 131; 231) der Zwischenkomponente (3; 103; 203) montiert ist.

10. Prothese nach einem der Ansprüche 1 bis 8, **dadurch gekennzeichnet, dass** das Zurückhalteorgan ein Monoblock mit der Zwischenkomponente ist.

11. Prothese nach einem der vorhergehenden Ansprüche, **dadurch gekennzeichnet, dass** das Zurückhalteorgan aus mindestens zwei Teilen gebildet wird.

12. Prothese nach einem der vorhergehenden Ansprüche, **dadurch gekennzeichnet, dass** die Humerus- oder Femurkomponente zweiteilig ist, wobei der Teil (11, 211), der dazu ausgelegt ist, im Markkanal des Humerus oder Femurs verankert zu werden, einen Sitz (11a) zum Aufnehmen eines mit dem Hals (13; 213) und der Gleitkufe (14; 214) einstückigen Metallstücks (17) definiert.

13. Prothese nach Anspruch 12, **dadurch gekennzeichnet, dass** das Metallstück (17) auf einer Achse (X₁₇) zentriert ist, die in Bezug auf die Längsachse (X₁₃) des Halses (13) versetzt (d) ist.

14. Prothese nach Anspruch 13, **dadurch gekennzeichnet, dass** der Sitz (11a) dazu ausgelegt ist, das Metallstück (17) in mindestens zwei Positionen aufzunehmen, in denen die zentrale Achse (X₁₇) des Metallstücks mit einer zentralen Achse (X₁₁) des Sitzes (11a) ausgerichtet ist, wobei die Winkelposition der Längsachse (X₁₃) des Halses (13) in Bezug auf die zentrale Achse (X₁₁) des Sitzes bei den beiden Positionen unterschiedlich ist.

15. Verfahren zur Montage einer Schulter- oder Hüftprothese nach Anspruch 1, umfassend eine Humerus- oder Femurkomponente, die eine konkave Artikulations-oberfläche aufweist, und eine Zwischenkomponente mit einer ersten und einer zweiten konvexen Artikulations-oberfläche, die dazu bestimmt sind, jeweils mit der konkaven Artikulationsoberfläche der Humerus- oder Femurkomponente und mit einer konkaven Glenoid- oder Acetabulum-Artikulationsoberfläche zusammenzuwirken, die natürlich ist oder sich an einer Glenoid- oder Acetabulumkomponente befindet, **dadurch gekennzeichnet, dass** es die folgenden Schritte umfasst:
- Einbringen eines Halses (13; 113; 213), der sich an der Humerus- (1; 201) oder Femurkomponente (101) befindet, in einen nicht-runden Durchgang (34; 134; 234), der von einem Organ (33; 133; 233) gebildet wird, das dazu bestimmt ist, Teil der Zwischenkomponente (3; 103; 203) zu sein;
Verlagern einer Gruppe, die von dem Hals, dem Organ und einer Gleitkufe (14; 114; 214), welche die konkave Artikulationsoberfläche der Humerus- oder Fe-murkomponente bildet, gebildet wird, bis die Gleitkufe gegen die erste konvexe Artikulationsoberfläche (S'₁) der Zwischenverbindung aufliegt, und
- Immobilisieren des Organs in Bezug auf einen Hauptteil (31; 131; 231) der Zwischenkomponente derart, dass ein Teil (14a, 114a, 214a) der Gleitkufe in einem Aufnahmevolumen (E), das zwischen dem Organ und der ersten konvexen Oberfläche definiert wird, zurückgehalten wird.

16. Verfahren nach Anspruch 15 zur Montage einer Prothese nach Anspruch 6, **dadurch gekennzeichnet, dass** man beim Verlagern der Gruppe (113, 114, 133, 213, 214, 233) die Gleitkufe (114; 214) in Bezug auf das Zurückhalteorgan (133; 233) derart orientiert, dass ihre größte Abmessung (X₁₁₄-X'₁₁₄ ; X₂₁₄-X'₂₁₄) im Großen und Ganzen senkrecht zu der größten Abmessung (X₁₃₄-X'₁₃₄ ; X₂₃₄-X'₂₃₄) des Durchgangs (134, 234) ist.

17. Verfahren nach Anspruch 15 zur Montage einer Prothese nach den Ansprüchen 7 und 8, **dadurch gekennzeichnet, dass** man beim Verlagern der Gruppe (113, 114, 133; 213, 214, 233) die Gleitkufe (114; 214) in Bezug auf das Zurückhalteorgan (133; 233) derart orientiert, dass die Zonen mit den kleinsten Dicken (e'₂, e'₄) des Teils (114a; 214a) der Gleitkufe, das in Bezug auf den Hals (113, 213) hervorspringt, sich jeweils mit den Teilen des Organs decken, die dazu bestimmt sind, die Zonen des Aufnahmevolumens (E) mit der kleinsten Dicke (e₁, e₂) zu definieren, während die Zonen mit den größten Dicken (e'₃, e'₄) des Teils sich jeweils mit den Teilen des Organs decken, die dazu bestimmt sind, die Zonen des Volumens mit den größten Dicken (e₃, e₄) zu definieren.

18. Verfahren zur Montage einer Schulter- oder Hüftprothese nach Anspruch 1, wobei die Prothese eine Humerus- oder Femurkomponente, die eine konkave Artiku-lationsoberfläche aufweist, und eine Zwischenkomponente mit einer ersten und einer zweiten konvexen Artikulationsoberfläche umfasst, die dazu bestimmt sind, jeweils mit der konkaven Artikulationsoberfläche der Humerus- oder Femurkomponente und mit einer konkaven Glenoid- oder Acetabulum-Artikulationsoberflä-che zusammenzuwirken, die natürlich ist oder sich an einer Glenoid- oder Acetabulumkomponente befindet, **dadurch gekennzeichnet, dass** es einen Schritt umfasst, bei dem die Gleitkufe (14; 114; 214) der Humerus- (1; 201) oder Femurkomponente (101) mit Kraft in den Durchgang (34; 134; 234), der von dem Zurückhalteorgan (33; 133; 233) gebildet wird, derart eingebracht wird, dass der Hals (13; 113; 213) in den Durchgang eingesetzt wird und dass ein Teil (14a ; 114a ; 214a) der Gleitkufe in einem Aufnahmevolumen (E) zurückgehalten wird, das zwischen dem Organ und der ersten konvexen Oberfläche (S'₁) der Zwischenverbindung (3; 103; 203) definiert wird.
